# EUROPEAN PATENT APPLICATION

(11) **EP 4 049 700 A1**
(43) Date of publication of application: **31.08.2022**
(21) Application number: 21315028.7
(22) Date of filing: 25.02.2021
(51) Int. Cl.: A61M 11/00, B05B 1/00, A61M 15/08

(54) **FLUID DISPENSING DEVICE**

(71) Applicant: SANOFI, 75008 Paris (FR)
(72) Inventor: The designation of the inventor has not yet been filed
(74) Representative: Sanofi Aventis Deutschland GmbH

(57) **Abstract**

The present disclosure relates to a a fluid dispensing device (10) comprising:
- a housing (11) sized to accommodate a container (20) filled with a liquid substance (21),
- the container (20) comprising a cavity (22) confined by a shell (23), the shell (23) comprising at least a flexible shell portion (24),
- a discharge assembly (30) comprising a first member (40) and a second member (50),
- the first member (40) comprising a hollow tubular section (41) in flow communication with the cavity (22), and
- the first member (40) and the second member (50) being movable relative to each other along a first direction to discharge a dose of the liquid substance (21) from the cavity (22).

## Description

### Description

The present disclosure relates to the field of fluid dispensing devices and in particular to fluid dispensing devices configured as nasal inhalers or configured as spray delivery devices. The disclosure further relates to spray delivery devices configured to dispense a fluid or a liquid substance by way of spraying or atomizing.

### Background

Fluid dispensing devices operable to atomize a liquid substance are as such known. Such devices typically comprise a nozzle or an orifice. Upon application of a force by a user to an actuation lever or a button the fluid is dispensed via the nozzle or orifice. Such devices may be arranged to dispense a single dose or such devices may be equipped with a container providing a reservoir for the fluid thus allowing and supporting the dispensing of several doses.

Fluid dispensing or spray delivery devices often comprise a pump actuator that has to be depressed by a user several times to achieve a first spray delivery. Typically, an initial spray delivery does not provide the required spray characteristics in terms of spraying or atomizing quality, amount of fluid dispensed or with regards to a desired spatial distribution of the spray. With most spray delivery devices or fluid dispensing devices the device has to be kept upright to avoid aspirating air into the pump. Aspiration of air into the pump may lead to a poor spray quality. With the known deficiencies of such fluid dispensing or spray delivery devices many users have learned to use such devices in a manner, wherein they repeatedly activate or conduct a delivery action until the spray is good enough or fulfills a desired quality criterium. Such scenarios of use may come along with a non-neglectible waste of the liquid substance to be dispensed.

It is therefore desirable to provide an improved fluid dispensing or spray delivery device that overcomes the shortcomings of solutions known so far. The fluid dispensing device should provide a reliable and highly reproducible spray characteristics. The fluid dispensing device should provide spray delivery of a liquid substance or atomizing of a liquid substance irrespective of its orientation. The fluid dispensing device should further provide a rather long-term usability of the liquid substance. It is a further aim to increase patient safety, both in terms of dosing accuracy and durability or stability of the liquid substance to be dispensed.

### Summary

In one aspect there is provided a fluid dispensing device. The fluid dispensing device may be implemented as a spray delivery device. It may provide discharging of a well-defined amount of a liquid substance, i.e. a dose of the liquid substance in atomized form. The dispensing device may be implemented as multi-dose dispensing device.

The fluid dispensing device comprises a housing sized to accommodate a container. The container is filled with a liquid substance. The liquid substance or successive doses thereof are intended to be dispensed by the fluid dispensing device. The container comprises a cavity. The cavity is confined by a shell. The shell comprises at least a flexible shell portion. The fluid dispensing device further comprises a discharge assembly. The discharge assembly comprises a first member and a second member. The first member comprises a hollow tubular section in flow communication with the cavity. The first member and the second member are movable relative to each other along a first direction to discharge a dose of the liquid substance from the cavity.

The shell that defines or confines the cavity of the container is at least in sections made of a flexible material. The flexible material, hence the at least one flexible shell portion supports and allows an elastic and/or plastic deformation of the shell and hence of the container when a portion of the liquid substance is withdrawn or expelled from the container. Contrary to containers comprising a rigid container structure, a rigid shell or a rigid barrel the flexible deformation capability of the shell avoids ingress of air, after an amount, e.g. a dose of the liquid substance has been withdrawn from the cavity.

Typically, the at least one flexible shell portion is flexibly deformable in response to a withdrawal of a portion of the liquid substance from the cavity in response to a negative pressure inside the cavity. The negative pressure inside the cavity may be caused by a movement of the first member and the second member relative to each other along the first direction. The first and the second member and hence the discharge assembly may be implemented as a pump. The pump may be manually depressed or actuated by a user. Hence, the movement of the first member relative to the second member may be manually inducible by a user of the fluid dispensing device. Repeated back and forth movement of the first member relative to the second member may repeatedly induce a negative pressure inside the cavity. With each negative pressure build up the flexible shell portion is configured to deform accordingly.

In effect, the at least one flexible shell portion of the shell serves to provide a pressure equilibrium of the pressure inside the cavity with regards to a surrounding pressure outside the cavity. Since the shell of the container comprises at least in sections a flexible shell portion ingress of air or other contaminants into the interior of the container and hence into the cavity can be effectively avoided. In this way a rather airtight and closed fluid dispensing device and fluid dispensing system can be provided.

Typically, the container may be entirely filled with the liquid substance. The cavity of the container may be gas-less. The cavity may be entirely filled with the liquid substance. It may be void of a gaseous substance. Withdrawal of at least a portion, e.g. of a dose of the liquid substance may then induce a deformation and a modification of the shape of the cavity, which tends to achieve a pressure equilibrium with the outer environment, hence with the surrounding of the cavity.

The flexible deformation capability of at least a portion of the shell and hence of at least a portion of the container enables the implementation of an air- or gas-tight and hence of a closed container exhibiting a reduced susceptibility for ingress of air or other contaminants. The container as a primary pack for a liquid substance comes along with a significantly reduced risk of loss of sterility, e.g. caused by external air ingress. Moreover, a rather closed and airtight container and/or a respective discharge assembly may allow storage of a preservative free formulation of the liquid substance inside the cavity. An airtight or closed system of first member, second member and the container may allow a direct use of oxygen sensitive formulations as a liquid substance inside the cavity.

In effect and according to a further example the discharge assembly and the container are sealed against ingress of air or other contaminants. This particularly applies during and/or after moving the first member relative to the second member along the first direction and/or along a second direction opposite the first direction. Typically, the first member and the second member are movable relative to each other in a sealed manner.

The movement between the first member and the second member may be configured to allow withdrawal of the liquid substance from the container but prevents ingress of air or other contaminants into the interior or cavity of the container.

According to a further example the second member comprises a hollow tubular section sized to slidably receive the hollow tubular section of the first member therein. Accordingly, the first member and the second member are at least in sections or at least partially arranged in a nested manner. The hollow tubular section of the second member and the hollow tubular section of the first member may be arranged coaxially. Typically, the hollow tubular section of the first member is slidably displaceable relative to the hollow tubular section of the second member along a central axis of the tubular section of the first member and a central axis of the tubular section of the second member. With the coaxial arrangement of tubular sections of the first member and of the second member, respective symmetry axes of the hollow tubular sections may coincide or at least partially overlap.

The nested arrangement of tubular sections of the first member and of the second member provide a rather space saving arrangement of first and second members. In this way, a rather compact discharge assembly can be provided. This allows to implement the fluid dispensing device as a handheld dispensing device that can be easy carried along by a user. Moreover, with tubular sections, there can be obtained a comparatively good and reliable sealing between the first and the second members.

According to a further example the tubular section of the first member comprises a tubular-shaped sidewall with at least one through opening. With some examples the tubular section of the first member comprises numerous through openings, e.g. equidistantly arranged along the circumference of the tubular-shaped sidewall. The through openings provide a flow communication between the interior of the tubular section of the first member and the outside of the tubular section of the first member.

As the first and the second members are subject to a movement relative to each other along the first direction, e.g. along the longitudinal axis of the respective tubular sections of the first and/or of the second member a negative pressure may be built up in a gap between the first member and the second member. Such a negative pressure may lead to a withdrawal of at least a portion of the liquid substance through the hollow tubular section of the first member and through the at least one through opening in the sidewall of the tubular section of the first member.

By providing numerous through openings in the sidewall of the tubular section of the first member the mass flow of the liquid substance through the hollow tubular section towards the second member can be increased. Accordingly, the volumetric efficiency of the fluid dispensing device can be improved.

With another example the tubular section of the first member is axially or longitudinally confined by a closed wall. In the present context, the axial direction, in particular an axial distal direction may coincide with the first direction. It may further coincide with the symmetry axis of at least one of the tubular section of the first member and the tubular section of the second member. The closed wall may comprise or may constitute an axial end face of the tubular section of the first member. In this way the interior of the hollow tubular section of the first member can be sealed and hermetically separated from the interior of the hollow tubular section of the second member.

The closed wall e.g. at a distal axial end of the tubular section of the first member may further improve a stability and mechanical rigidity of the tubular section of the first member.

According to another example the tubular section of the first member is sheathed by a flexible tubular shaped seal. The tubular shaped seal may comprise a flexible material, such as a natural or synthetic rubber material. The flexible tubular shaped seal may be press fitted on the outside surface of the tubular section of the first member. It may tightly enclose the tubular section of the first member. In particular, the flexible tubular shaped seal may cover the at least one through opening of the tubular-shaped sidewall of the first member. The tubular shaped seal is open towards the distal end, it is void of an axial front face. In other words the flexible tubular shaped seal does not cover the closed wall of the tubular section of the first member. A longitudinal or distal end of the tubular shaped seal may terminate at a predefined distance from the axial or distal end of the tubular section of the first member. With some examples the longitudinal distal end of the flexible tubular shaped seal may flush with a longitudinal distal end of the sidewall of the tubular section of the first member.

In any case the tubular shaped seal covers and seals the at least one through opening extending through the tubular-shaped sidewall of the tubular section of the first member.

With some examples the flexible tubular shaped seal comprises an initial shape before it is mounted on the outside surface of the tubular shaped sidewall. In the initial or unbiased shape, an inner diameter or inner cross-section of the tubular shaped seal may be slightly smaller than an outer diameter or outer cross-section of the tubular-shaped sidewall. In this way and when mounted or arranged on the outside of the tubular-shaped sidewall of the tubular section of the first member the flexible tubular shaped seal has to be elastically deformed and has to be slightly elastically expanded in radial direction. Such an at least slight elastic widening or deformation of the flexible tubular shaped seal provides a tight fit between the tubular -shaped sidewall and the tubular shaped seal. In this way, the at least one through opening of the tubular-shaped sidewall of the first member can be effectively sealed against ingress of gaseous or liquid substances.

Closing and sealing of the at least one through opening of the tubular-shaped sidewall by an elastic tubular shaped seal has the benefit that the interior of the hollow tubular section of the first member can be effectively sealed against ingress of air or other contaminants in case that there should evolve a negative pressure inside the hollow tubular section of the first member and hence inside the cavity compared to the surrounding pressure.

With other scenarios, wherein an outside pressure is smaller than an inside pressure of the cavity there will typically arise a respective suction effect by way of which a respective portion of the liquid substance may escape through the at least one through opening and through the sealing engagement between the outside surface of the tubular-shaped sidewall and an inside surface of the flexible tubular shaped seal. In such a configuration or situation a respective portion, e.g. a dose of the liquid substance may escape through the hollow tubular section of the first member, through the at least one through opening of the tubular-shaped sidewall of the first member, through an interface between an outside surface of the tubular-shaped sidewall and an inside surface of the flexible tubular shaped seal and into the interior of the hollow tubular section of the second member. In this way at least a dose of the liquid substance can be transported or delivered from the cavity into an intermediate space or gap provided between the first member and the second member.

According to a further example the tubular section of the first member is in engagement with the tubular section of the second member. This sealing engagement can be provided with and/or by the flexible tubular shaped seal surrounding the tubular section of the first member. Alternatively, the sealing engagement between the tubular section of the first member and the tubular section of the second member can be provided by another or by a further seal, e.g. provided on an inside surface of the tubular section of the second member.

Providing of a sealing engagement between the tubular section of the first member and the tubular section of the second member by the flexible tubular shaped seal surrounding or sealing the first member is beneficial to reduce the total number of parts for assembly of the fluid dispensing device and the discharge assembly. In this way, the number of components for assembling the discharge assembly can be reduced. Manufacturing costs and be saved accordingly. Here, the flexible tubular shaped seal may provide a twofold or double function. The flexible tubular shaped seal effectively closes and seals the at least one through opening extending through the tubular-shaped sidewall of the first member. In addition, the tubular seal further provides an effective seal, e.g. an air-tight seal between the interior of the second member and an exterior of the first member.

By way of a sealing engagement between the tubular section of the first member and the tubular section of the second member a dispensing chamber can be provided in a gap or gap space between the outside of the tubular section of the first member and an inside of the hollow tubular section of the second member. The gap space may be located axially between the axial distal end of the first member and an inside of the second member. The gap space may be confined in longitudinal or axial direction by the first member and by the second member, respectively. In circumferential direction the gap space may be confined by the sidewall of the tubular section of the second member.

Particularly, the flexible tubular shaped seal surrounding and enclosing the tubular -shaped sidewall of the tubular section of the first member provides and/or constitutes an inlet valve for a unidirectional flow of the liquid substance from the cavity into the dispensing chamber. The flexible tubular shaped seal, e.g. implemented as a flexible hose, provides a passive seal and closing mechanism. Such a self-closing seal or unidirectional flow valve only comprises a minimized tendency to allow air into the device through the inlet valve and provides only a minimum leaking of a gaseous or liquid substance located inside the dispensing chamber back into the cavity of the container.

Moreover, the inlet valve comprising the tubular section of the first member sheathed by the flexible tubular shaped seal is relatively shock insensitive. It provides an effective sealing irrespective of the influence of gravitation. The sealing capability is not influenced by the orientation of the device.

The improved sealing capability of the inlet valve provides a high degree of maintaining a spray quality for all doses in a multidose fluid dispensing device.

According to a further example the hollow tubular section of the second member comprises or constitutes a dispensing chamber that is axially confined by the tubular section of the first member. The size of the dispensing chamber is variable by moving the first member relative to the second member. The dispensing chamber may be of tubular shape. It may be confined in radial direction by a sidewall portion of the hollow tubular section of the second member. With some examples, the dispensing chamber is axially or longitudinally confined by respective end walls of the hollow tubular section of the second member and of the tubular section of the first member.

With some examples the dispensing chamber is axially and longitudinally confined in distal direction by an end wall of the hollow tubular section of the second member. The dispensing chamber may be confined in proximal direction by the first member, in particular by the tubular section of the first member. With some examples the closed wall axially confining the tubular section of the first member in distal direction forms a proximal end face of the dispensing chamber of the fluid dispensing device. The closed wall may coincide with or may constitute a distal end of the first member

With some examples the volume or size of the dispensing chamber is reduced as the first member is moved relative to the second member along a first or distal direction. A movement of the first member relative to the second member in the opposite direction, hence along a longitudinal or axial proximal direction may lead to an increase of the volume or size of the dispensing chamber.

A movement of the first member relative to the second member in distal direction, which is accompanied by a reduction of the volume or size of the dispensing chamber may lead to a dispensing of the liquid substance contained inside the dispensing chamber. The distally directed movement of the first member relative to the second member may expel a dose of the medicament contained inside the dispensing chamber. Here, the entirety or almost the entirety of the liquid substance contained in the dispensing chamber may be expelled through an orifice or through opening provided in an end wall of the hollow tubular section of the second member. The end wall may be a distal end wall and may confine the dispensing chamber in distal direction.

A movement of the first member relative to the second member in the opposite direction, hence in proximal direction, which is accompanied by an increase of the volume of the dispensing chamber may lead to the build-up of a negative pressure inside the dispensing chamber compared to the pressure inside the cavity and/or compared to the surrounding pressure.

Build-up of a negative pressure inside the dispensing chamber leads to a withdrawal of liquid substance from the cavity of the container through the hollow tubular section of the first member, through the at least one through opening of the tubular-shaped sidewall of the first member, which is accompanied by an at least slight radially directed flexible deformation of the flexible tubular shaped that seals the surrounding or the outer circumference of the tubular section of the first member.

According to a further example the second member comprises an outlet arranged at a longitudinal, e.g. at an axial distal end of the hollow tubular section of the second member. The outlet is located at a portion of the hollow tubular section of the second member that faces away from the first member. The outlet is further sealed by an outlet valve. The outlet valve may be implemented as a one-way valve, as check valve or as a duckbill valve. The outlet may be located in a radial central portion of the hollow tubular section of the second member. The outlet valve may entirely cover the outlet of the second member.

The outlet valve is configured to allow and to support expelling of a liquid substance from the dispensing chamber that is confined by an outside of the first member and an inside of the tubular section of the second member. When implemented as a check valve, the outlet valve prevents ingress of any gaseous substances or other contaminants from outside the outlet into the interior of the hollow tubular section of the second member.

In this way and when the first member is subject to a proximally directed movement relative to the second member the outlet valve or check valve effectively prevents ingress of any substances from outside and supports the build-up of a negative pressure in the dispensing chamber provided between the inside of the hollow tubular section of the second member and the outside of the tubular section of the first member.

According to a further example the shell of the container comprises a flexible foil or is made of a flexible foil. The shell may be in sealed engagement with the first member. The first member may be connected to an outlet of the flexible foil. The first member, typically made of a rigid material, such as an injection molded plastic material may reach or extend through the shell and may protrude from an outside surface of the shell with its hollow tubular section.

With some examples it may be the second member of the discharge assembly that is connected to the shell and that may penetrate the shell. Also here, it may be the hollow tubular section of the second member that may reach through or extend through an orifice in the shell and that may protrude from an outside surface of the shell. With any example, at least one of the first member and the second member may be welded or bonded to the shell or flexible foil in order to provide an airtight seal between the discharge assembly and the container. In this way, ingress of air or any other contaminants into the cavity of the container can be effectively prevented.

With some examples the container comprises a flexible bag forming or constituting the shell and confining the cavity. The flexible bag or the flexible shell can be entirely made of a flexible foil. In this way, the container may comprise a high degree of flexibility. It may easily conform to a desired construction space inside a fluid dispensing device. A container of flexible or deformable shape allows and supports a function specific design of the fluid dispensing device.

According to a further example an interior of the hollow tubular section of the first member is in flow communication with a dip tube extending into the cavity of the container. The dip tube may be of tubular shape or of any other hollow shape. The dip tube may be directly connected to the hollow tubular section of the first member. The dip tube may comprise or may constitute a longitudinal extension of the first member.

Typically, the dip tube extends in proximal direction from the first member and into the cavity of the container. The dip tube may terminate at a predefined portion inside the container. With some examples the dip tube is of longitudinal shape. The dip tube may be of rather straight shape. The dip tube may comprise a hollow tubular shape. The dip tube may comprise an end section or end portion remote from the first member. This particular end section or end portion may be located at or may abut against an inside surface of a portion of the shell located opposite to the discharge assembly. In this way, the dip tube may reach through or traverse the entirety of the container. The dip tube may thus provide and support a complete emptying of the liquid substance from the container.

According to a further example the dip tube comprises a first longitudinal end and a second longitudinal end. The second longitudinal end is located opposite the first longitudinal end. The first longitudinal end is connected to the first member. The first longitudinal end may be integrally formed with the first member. Hence, the dip tube and the first member may be made of the same material, e.g. of an injection moldable plastic material. The material the dip tube and/or the first member are made of is typically inert with regards to the liquid substance contained inside the cavity of the container. With some examples, at least one of the first member, the second member and the dip tube is made of a cyclic olefin copolymer (COC) material.

A direct connection of the dip tube with the first member provides an effective flow communication between the interior of the first member and the cavity. By way of the dip tube, transportation of the liquid substance located inside the cavity at a portion or location remote from the first member can be effectively provided.

According to another example the dip tube is pressure resistant at least with regard to the first or longitudinal direction. The second longitudinal end of the dip tube is connected to a base or it is operably engaged with a base. It may be in abutment with the base. The base is movable relative to at least one of the housing and the second member along the first direction. With some examples the base is actuatable by a user from outside the housing of the fluid dispensing device. A movement of the base relative to the housing and/or relative to the second member may be directly induced by a user of the fluid dispensing device, e.g. in order to trigger or to control a liquid dispensing operation. Typically, the base itself is made of a rigid material, e.g. of an injection moldable plastic material. The base may be either directly connected to the dip tube or the base may be in abutment with the dip tube such that a movement of the base along the first direction relative to at least one of the housing and the second member transfers to a respective movement of the dip tube relative to the housing and/or relative to the second member. The base may be implemented as a user-operable actuator or trigger.

With a pressure resistant implementation of the dip tube the first longitudinal end of the dip tube can be fixed to the first member. In this way a movement of the base relative to at least one of the housing and the second member transfers onto the dip tube and hence onto the first member. Accordingly, a movement of the base as induced by the user of the device can be transferred into a movement of the first member relative to at least one of the housing and the second member.

With some examples the base is movably arranged on or inside the housing along the first direction and along a second direction which is opposite to the first direction. With some examples the base may be movable relative to the housing along another direction, e.g. a direction extending at a certain angle relative to the first direction. With such examples there may be provided a kind of a mechanical transmission between the base and the first member such that a movement of the base along a predefined direction transfers into a required movement of the first member along the first direction and/or along the second direction opposite to the first direction.

The base may be slidably displaceable relative to the housing and/or relative to the second member. It may be slidable along the first and/or the second direction. With some examples the base may be pivotable relative to the housing and/or relative to the second member. With a respective transmission that may be provided by a crank arm or the like mechanical transmission means a pivoting motion of the base may be transferable into a longitudinal sliding displacement of the first member relative to the second member via the dip tube or via some other mechanical transmission component.

With some examples the base may be located outside the container. It may be in an effective abutment with the dip tube located inside the cavity of the container. Here, a movement of the base may transfer through the flexible shell of the container towards and onto the dip tube. With other examples the base may form part of the container. Hence, the base may contribute to the shell of the container. Here, the base may form a portion of the shell of the container, e.g. a rigid portion of the shell of the container. The base may be connected and sealed with at least one or several flexible portions, such as a flexible foil portions of the shell. With other examples the base may be located inside the cavity of the container. It may be surrounded by the shell of the container. Since the shell of the container is flexible the base may be actuatable or depressible by a user from outside the housing of the fluid dispensing device. Here, a user may apply a certain actuation force onto a dedicated and accessible portion of the shell of the container that covers the base.

According to a further example the dip tube comprises a sidewall perforated by numerous through openings. In this way a complete emptying of the container can be provided. The through openings may be provided all over the circumference or sidewall of the dip tube. They may be arranged regularly along the sidewall of the dip tube or irregularly along or across the sidewall of the dip tube. Through openings may be provided at the first longitudinal end, at the second longitudinal end and/or in sections located between the first and second longitudinal ends of the dip tube. Since the container is flexibly deformable and since the container may be filled with the liquid substance in a gaseous-free manner with the liquid substance ingress of air or other contaminants into the cavity is effectively avoided. With the present fluid dispensing device or discharge assembly an effective withdrawal of the liquid substance can be obtained in any orientation of the fluid dispensing device or of its container.

Moreover and since ingress of air or other contaminants into the discharge assembly is effectively avoided, the fluid dispensing device may provide a highly reproducible spray and atomizing characteristics even when actuated initially or for a first time. Hence, a user may be no longer obliged to actuate the discharge assembly multiple times until a spray of desired quality will be delivered by the discharge assembly or by the fluid dispensing device.

According to a further example the fluid dispensing device comprises a mechanical biasing member. The mechanical biasing member is operably engaged with two of the first member, the second member and the housing. The first member is movable relative to the second member along the first direction against a restoring force provided by the mechanical biasing member. With some examples the mechanical biasing member is operably engaged with the first member and the housing of the fluid dispensing device. Here, the second member of the discharge assembly may be fixed to the housing. A user-induced movement of the first member relative to the housing against the action of the mechanical biasing member may lead to the storage of mechanical energy in the mechanical biasing member. When a user actuation has terminated the mechanical biasing member may then serve to return the first member relative to the second member and relative to the first housing into an initial or idle position or configuration.

With another example the mechanical biasing member is operably engaged between the first member and the second member of the discharge assembly. Also here, a user-induced movement of the first member relative to the second member may be accompanied by storing of mechanical energy in or by the mechanical biasing member. A respective amount of mechanical energy may be released as soon as a user-induced action has terminated. Here, one of the first member and the second member may be rigidly connected or fixedly attached to the housing while the other component of the first member and the second member is reversibly movable against and under the action of the mechanical biasing member, respectively.

With another example the mechanical biasing member is operably engaged with the housing and the second member of the discharge assembly. Here, it may be the first member that is fixedly attached to the housing. A user induced movement of the second member relative to the first member and/or relative to the housing is then typically accompanied by a respective storage of mechanical energy in the mechanical biasing member.

In some cases, the mechanical biasing member serves to return the first member and the second member into an initial configuration. Starting from the initial configuration a user may manually actuate one of the first member and the second member in order to induce a relative movement therebetween and/or to control a dispensing of a dose of the liquid substance.

With other examples the mechanical biasing member may be mechanically biased after delivery of a dose of the liquid substance and when the first and the second members return into an initial configuration. Then and as long as the fluid dispensing device is in an initial configuration the mechanical biasing member may be pre-loaded or pre-biased. Here, the fluid dispensing device may be equipped with a user actuatable trigger, which when actuated by the user serves to release mechanical energy from the mechanical biasing member. The mechanical energy released by the mechanical biasing member can be effective to displace the first member relative to the second member for dispensing a dose of the liquid medicament from the cavity of the container.

According to a further example the mechanical biasing member comprises a compression spring having a first end and a second end. The second end is located opposite the first end. The first end is in abutment or is engaged with the housing. The second end is mechanically engaged or is in abutment with the first member or the second member. With a compression spring, a rather robust and failure safe mechanical biasing member can be provided. The compression spring may comprise a helical shape. The compression spring can be easily adapted to predefined construction space provided by the housing and the discharge assembly. The compression spring may be in direct mechanical engagement or mechanical abutment with the base. With some examples the compression spring may be located between an abutment portion of the housing and a respective abutment portion of the base. In this way, the user actuatable base may be depressible or movable against the action of the compression spring either for preloading the compression spring or for and during dispensing of the liquid substance via the discharge assembly.

With another example the compression spring is in mechanical abutment or engagement with the first member and with the second member, respectively. Here, a first end of the compression spring may be in mechanical abutment or engagement with the first member and the opposite end, hence the second end of the compression spring may be in mechanical abutment or engagement with the second member. With this example the dip tube rigidly connected to the first member may be only in mechanical abutment with the base. It does not have to be fixed to the base. Here, it will be sufficient when the base is configured and operable to apply a distally directed pressure onto the dip tube, which transfers into a distally directed movement of the first member relative to the second member. A return motion into an initial configuration may be effectuated by the compression spring. Here, the compression spring may serve to exert a proximally directed driving force onto the first member thereby urging the first member, the dip tube and the base in proximal direction relative to the second member.

According to a further example the container prefilled with the liquid substance is arranged inside the housing. The container may be arranged inside the housing via at least one of the first member and the second member. The container may be exchangeable and/or displaceably arranged inside a container receptacle provided by the housing. Typically, the entirety of the container is located inside the housing. In this way, the container is invisible from outside the housing. Typically, the first member and the second member are made of rigid plastic materials. With some examples, at least one of the first member and the second member is in liquid proof sealing engagement with the shell of the container. At least one of the first member and the second member provides a mount for arranging and fixing the container inside the housing of the fluid dispensing device. In this way, at least one of the first member and the second member does not only contribute to the discharge assembly but further allows and supports a proper and well-defined handling, arranging and/or fixing of the container inside the housing of the fluid dispensing device.

In another aspect the disclosure relates to a container for a fluid dispensing device. The container comprises a cavity confined by a shell. The shell comprises at least a flexible shell portion. The container further comprises a first member. The first member is at least partially located outside the cavity and comprises a hollow tubular section in flow communication with the cavity. In particular, an inside of the hollow tubular section of the first member is in flow communication with the cavity.

The first member may be implemented as a first member of a discharge assembly of the fluid dispensing device as described above. With the present example the first member belongs to the container and is a component of the container. The first member comprises a rigid or solid structure. The first member may be manufactured from a rigid material, e.g. from an injection moldable plastic material.

The first member may provide a well-defined mounting, assembly and/or fixing of the container inside a housing of a fluid dispensing device as described above. Moreover, the first member may provide and support a well-defined withdrawal of the liquid substance from the cavity of the container.

With some examples the cavity of the container is sealed. It may be sealed airtight or leakage-proof with regards to liquid and/or gaseous substances. The cavity may be filled with a liquid substance, e.g. containing a pharmaceutical active component or ingredient.

According to a further example the first member of the container is slidably received inside a hollow tubular section of a second member. The second member forms or comprises a dispensing chamber axially confined by the tubular section of the first member. With some examples the second member is a component of the container. Hence, the container comprises the cavity as confined by the shell. The container further comprises the first member and the second member. With other examples the second member belongs to a fluid dispensing device or to a discharge assembly of such a fluid dispensing device as described above. The container may be detachably connected to the fluid dispensing device by detachably connecting or detachably fixing the first member to the second member.

According to a further example the second member comprises an outlet at a longitudinal end of the hollow tubular section of the second member that faces away from the first member. The outlet may adjoin the dispensing chamber and may provide a fluid dispensing outlet for the dispensing chamber. The liquid substance located inside the dispensing chamber may be expelled from the dispensing chamber through the outlet, e.g. by reducing the volume of the dispensing chamber through a movement of the first member relative to the second member. By moving the first member relative to the second member a liquid expelling mechanism can be provided.

According to another example the outlet of the second member is sealed by an outlet valve. The outlet valve may be implemented as a check valve or may comprise a check valve. The outlet valve supports and allows dispensing of the liquid substance from the dispensing chamber towards and into the outlet, thereby allowing and supporting a spray discharge of the liquid substance while prevent ingress of gaseous substances, e.g. of air or other contaminants into the dispensing chamber via the outlet. In this way a rather closed airtight container can be provided that allows and supports storing of the liquid substance inside the cavity without any preservatives.

Also, liquid substances comprising an oxygen sensitive formulation may be easily stored inside the cavity even over a comparatively long storage time. Since the cavity comprises at least a flexible shell portion withdrawal of a portion of the liquid substance from the cavity comes along with a respective flexible deformation of the flexible shell portion. Typically and when the flexible shell portion is plastically deformable, the respective flexible shell portion does not tend to return into an initial configuration but remains in the squeezed or volume reduced configuration.

According to an example the hollow tubular section of the first member is axially confined by a closed wall fixed to the tubular-shaped sidewall or integrally formed with the tubular shaped member. The closed wall adjoins and confines the dispensing chamber. The closed wall of the tubular section of the first member effectively seals the dispensing chamber in a longitudinal proximal direction. By displacing the first member relative to the second member, e.g. in or along a first or distal direction the size or volume of the dispensing chamber can be successively reduced, thus leading to a pressure build-up inside the dispensing chamber, thereby inducing a dispensing or escape of the liquid substance from the dispensing chamber via the outlet and/or via the outlet valve or check valve.

According to a further example the hollow tubular section of the first member is sheathed by a flexible tubular shaped seal. The flexible tubular shaped seal extends around an outer circumference of the hollow tubular section and/or around the outer circumference of the sidewall of the hollow tubular section. Typically, the flexible tubular shaped seal covers, e.g. entirely covers the at least one through opening provided in the sidewall of the hollow tubular section of the first member. In this way, the inside of the hollow tubular section of the first member can be effectively sealed from the dispensing chamber.

The tubular shaped seal covering the at least one through opening that extends through the tubular-shaped sidewall of the first member forms or constitutes an inlet valve for the dispensing chamber. The inlet valve may comprise a kind of a check valve or duckbill valve. It supports a unidirectional flow of a liquid substance from the interior of the hollow tubular section of the first member towards the interior of the second member and/or towards or into the dispensing chamber but prevents a flow of a liquid substance in the opposite direction. Typically, the tubular shaped seal is tightly fitted around the outer circumference of the tubular section of the first member. In this way, uncontrolled ingress of impurities, contaminants or of gaseous substances into the interior of the hollow tubular section of the first member can be effectively prevented. In this way, the container is hermetically sealed and provides a closed primary packaging that allows and supports a long-term storage even of preservative free formulations as a liquid substance inside the cavity.

According to a further example the shell is connected to or is integrally formed with the second member. Accordingly, the container comprises the cavity as confined or defined by the shell, the first member and the second member. The second member can be made of a rigid material, e.g. an injection moldable plastic material.

According to a further example the shell is connected to or is integrally formed with the first member. With some examples, a first portion of the shell may be connected to or may be integrally formed with the first member. A second portion of the shell, separated from the first portion of the shell may be connected to or may be integrally formed with the second member. In this way, a highly integrated container for long-term storage of a liquid substance can be provided. At least one of the first member and the second member may be further used to couple, to connect or to engage the container with a housing of the fluid dispensing device and/or with a discharge assembly of a fluid dispensing device.

According to another example the shell comprises a first longitudinal end and a second longitudinal end. The second longitudinal end is located opposite to the first longitudinal end. At least one of the first member and the second member is connected to or is integrally formed with at least one of the first longitudinal end and the second longitudinal end. With some examples, at least one of the first member and the second member is operably engaged with at least one of the first longitudinal end and the second longitudinal end.

With an example it may be the second member that is connected to or that is integrally formed with a second longitudinal end of the shell. Here, the first member may be operably engaged with an opposite longitudinal end of the shell and hence of the container.

Here, the first member or a longitudinal extension thereof may extend through the cavity as defined by the shell. It may traverse the cavity from the first longitudinal end towards the second longitudinal end. Here, a longitudinal extension of the first member, e.g. in form of a longitudinal dip tube may be connected to the first member and may traverse the cavity of the container from the first member, which is typically in a nested or interleaved arrangement with the second member, to the second longitudinal end of the shell. Here, a first end of the dip tube may be connected to the first member and a second longitudinal end of the dip tube may be operably engaged or may be connected to the second longitudinal end of the shell.

According to another example the hollow tubular section of the second member protrudes outwardly from the shell. Here, the second member may protrude outwardly from the first longitudinal end of the shell. The outwardly protruding second member provides an easy and intuitive handling of the container, particularly in terms of arranging the container and fixing the container inside a housing of a fluid dispensing device.

According to another example the first member is in flow connection with a dip tube extending into the cavity of the container. The dip tube may extend into the cavity and may provide a complete emptying of the cavity irrespective of the orientation of the cavity. Typically, the dip tube is perforated. The dip tube comprises a hollow tubular structure with numerous perforations or through openings extending through the sidewall of the dip tube. This allows and provides ingress of a liquid medium or of the liquid substance into the interior of the dip tube at almost any longitudinal position of the dip tube. The hollow interior of the dip tube may be in direct flow communication with the hollow tubular section of the first member. In this way, and an unobstructed transport of a liquid substance through the through opening in the sidewall of the dip tube, into the interior of the dip tube and further into the interior of the hollow tubular section of the first member can be provided.

According to a further example the dip tube is pressure resistant or pressure stable at least with regard to the longitudinal direction of the shell. The dip tube further comprises a first longitudinal end and a second longitudinal end. The second longitudinal end is located opposite the first longitudinal end. The first longitudinal end is connected to the first member and the second longitudinal end is connected to or is in mechanical engagement with an end section of the shell of the cavity or with a base of a fluid dispensing device.

According to another example the base is connected to or is integrally formed with the second longitudinal end of the shell. With some examples the base may belong to the fluid dispensing device. The base may be displaceable against the action of the mechanical biasing member relative to the housing of the fluid dispensing device and/or relative to the second member.

Typically, the container as described herein is particularly dedicated and directly usable with a fluid dispensing device as described herein. The container may be manufactured and commercially distributed in connection with the fluid dispensing device. Hence, there may be provided a dispensing system comprising a fluid dispensing device as described above and equipped with a container as described above readily assembled inside the housing of the fluid dispensing device. Here, the container may be prefilled with the liquid substance, e.g. a medicament or a liquid substance comprising at least one pharmaceutically active component. With some examples of a prefilled container, the entire fluid dispensing device may be configured as a disposable device which is intended to become discarded in its entirety after use of the liquid substance provided inside the cavity of the container.

With other examples there is provided a dispensing system with a fluid dispensing device and with a number of containers to be assembled therein. Here, the container may be removably arranged or removably fixed inside the housing of the fluid dispensing device: Once the liquid substance inside the cavity has been used up or entirely dispensing an empty container may be replaced by another prefilled container.

Generally, the scope of the present disclosure is defined by the content of the claims. The injection device is not limited to specific embodiments or examples but comprises any combination of elements of different embodiments or examples. Insofar, the present disclosure covers any combination of claims and any technically feasible combination of the features disclosed in connection with different examples or embodiments.

In the present context the term 'distal', 'distal direction' or 'distal end' relates to an end of the injection device that faces towards a dispensing end of the device and/or to application site of a person or of an animal. The term 'proximal', 'proximal direction' or 'proximal end' relates to an opposite end of the injection device, which is furthest away from an application site of a person or of an animal.

The terms "drug" or "medicament" are used synonymously herein and describe a pharmaceutical formulation containing one or more active pharmaceutical ingredients or pharmaceutically acceptable salts or solvates thereof, and optionally a pharmaceutically acceptable carrier. An active pharmaceutical ingredient ("API"), in the broadest terms, is a chemical structure that has a biological effect on humans or animals. In pharmacology, a drug or medicament is used in the treatment, cure, prevention, or diagnosis of disease or used to otherwise enhance physical or mental well-being. A drug or medicament may be used for a limited duration, or on a regular basis for chronic disorders.

As described below, a drug or medicament can include at least one API, or combinations thereof, in various types of formulations, for the treatment of one or more diseases. Examples of API may include small molecules having a molecular weight of 500 Da or less; polypeptides, peptides and proteins (e.g., hormones, growth factors, antibodies, antibody fragments, and enzymes); carbohydrates and polysaccharides; and nucleic acids, double or single stranded DNA (including naked and cDNA), RNA, antisense nucleic acids such as antisense DNA and RNA, small interfering RNA (siRNA), ribozymes, genes, and oligonucleotides. Nucleic acids may be incorporated into molecular delivery systems such as vectors, plasmids, or liposomes. Mixtures of one or more drugs are also contemplated.

The drug or medicament may be contained in a primary package or "drug container" adapted for use with a drug delivery device. The drug container may be, e.g., a cartridge, syringe, reservoir, or other solid or flexible vessel configured to provide a suitable chamber for storage (e.g., short- or long-term storage) of one or more drugs. For example, in some instances, the chamber may be designed to store a drug for at least one day (e.g., 1 to at least 30 days). In some instances, the chamber may be designed to store a drug for about 1 month to about 2 years. Storage may occur at room temperature (e.g., about 20°C), or refrigerated temperatures (e.g., from about - 4°C to about 4°C). In some instances, the drug container may be or may include a dual-chamber cartridge configured to store two or more components of the pharmaceutical formulation to-be-administered (e.g., an API and a diluent, or two different drugs) separately, one in each chamber. In such instances, the two chambers of the dual-chamber cartridge may be configured to allow mixing between the two or more components prior to and/or during dispensing into the human or animal body. For example, the two chambers may be configured such that they are in fluid communication with each other (e.g., by way of a conduit between the two chambers) and allow mixing of the two components when desired by a user prior to dispensing. Alternatively or in addition, the two chambers may be configured to allow mixing as the components are being dispensed into the human or animal body.

The drugs or medicaments contained in the drug delivery devices as described herein can be used for the treatment and/or prophylaxis of many different types of medical disorders. Examples of disorders include, e.g., diabetes mellitus or complications associated with diabetes mellitus such as diabetic retinopathy, thromboembolism disorders such as deep vein or pulmonary thromboembolism. Further examples of disorders are acute coronary syndrome (ACS), angina, myocardial infarction, cancer, macular degeneration, inflammation, hay fever, atherosclerosis and/or rheumatoid arthritis. Examples of APIs and drugs are those as described in handbooks such as Rote Liste 2014, for example, without limitation, main groups 12 (antidiabetic drugs) or 86 (oncology drugs), and Merck Index, 15th edition.

Examples of APIs for the treatment and/or prophylaxis of type 1 or type 2 diabetes mellitus or complications associated with type 1 or type 2 diabetes mellitus include an insulin, e.g., human insulin, or a human insulin analogue or derivative, a glucagon-like peptide (GLP-1), GLP-1 analogues or GLP-1 receptor agonists, or an analogue or derivative thereof, a dipeptidyl peptidase-4 (DPP4) inhibitor, or a pharmaceutically acceptable salt or solvate thereof, or any mixture thereof. As used herein, the terms "analogue" and "derivative" refers to a polypeptide which has a molecular structure which formally can be derived from the structure of a naturally occurring peptide, for example that of human insulin, by deleting and/or exchanging at least one amino acid residue occurring in the naturally occurring peptide and/or by adding at least one amino acid residue. The added and/or exchanged amino acid residue can either be codable amino acid residues or other naturally occurring residues or purely synthetic amino acid residues. Insulin analogues are also referred to as "insulin receptor ligands". In particular, the term "derivative" refers to a polypeptide which has a molecular structure which formally can be derived from the structure of a naturally occurring peptide, for example that of human insulin, in which one or more organic substituent (e.g. a fatty acid) is bound to one or more of the amino acids. Optionally, one or more amino acids occurring in the naturally occurring peptide may have been deleted and/or replaced by other amino acids, including non-codeable amino acids, or amino acids, including non-codeable, have been added to the naturally occurring peptide. Examples of insulin analogues are Gly(A21), Arg(B31), Arg(B32) human insulin (insulin glargine); Lys(B3), Glu(B29) human insulin (insulin glulisine); Lys(B28), Pro(B29) human insulin (insulin lispro); Asp(B28) human insulin (insulin aspart); human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Examples of insulin derivatives are, for example, B29-N-myristoyl-des(B30) human insulin, Lys(B29) (N- tetradecanoyl)-des(B30) human insulin (insulin detemir, Levemir^{®}); B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-gamma-glutamyl)-des(B30) human insulin, B29-N-omega-carboxypentadecanoyl-gamma-L-glutamyl-des(B30) human insulin (insulin degludec, Tresiba^{®}); B29-N-(N-lithocholyl-gamma-glutamyl)-des(B30) human insulin; B29-N-(ω-carboxyheptadecanoyl)-des(B30) human insulin and B29-N-(ω-carboxyheptadecanoyl) human insulin.

Examples of GLP-1, GLP-1 analogues and GLP-1 receptor agonists are, for example, Lixisenatide (Lyxumia^{®}), Exenatide (Exendin-4, Byetta^{®}, Bydureon^{®}, a 39 amino acid peptide which is produced by the salivary glands of the Gila monster), Liraglutide (Victoza^{®}), Semaglutide, Taspoglutide, Albiglutide (Syncria^{®}), Dulaglutide (Trulicity^{®}), rExendin-4, CJC-1134-PC, PB-1023, TTP-054, Langlenatide / HM-11260C (Efpeglenatide), HM-15211, CM-3, GLP-1 Eligen, ORMD-0901, NN-9423, NN-9709, NN-9924, NN-9926, NN-9927, Nodexen, Viador-GLP-1, CVX-096, ZYOG-1, ZYD-1, GSK-2374697, DA-3091, MAR-701, MAR709, ZP-2929, ZP-3022, ZP-DI-70, TT-401 (Pegapamodtide), BHM-034. MOD-6030, CAM-2036, DA-15864, ARI-2651, ARI-2255, Tirzepatide (LY3298176), Bamadutide (SAR425899), Exenatide-XTEN and Glucagon-Xten.

An example of an oligonucleotide is, for example: mipomersen sodium (Kynamro^{®}), a cholesterol-reducing antisense therapeutic for the treatment of familial hypercholesterolemia or RG012 for the treatment of Alport syndrom.

Examples of DPP4 inhibitors are Linagliptin, Vildagliptin, Sitagliptin, Denagliptin, Saxagliptin, Berberine.

Examples of hormones include hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, and Goserelin.

Examples of polysaccharides include a glucosaminoglycane, a hyaluronic acid, a heparin, a low molecular weight heparin or an ultra-low molecular weight heparin or a derivative thereof, or a sulphated polysaccharide, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium. An example of a hyaluronic acid derivative is Hylan G-F 20 (Synvisc^{®}), a sodium hyaluronate.

The term "antibody", as used herein, refers to an immunoglobulin molecule or an antigen-binding portion thereof. Examples of antigen-binding portions of immunoglobulin molecules include F(ab) and F(ab')2 fragments, which retain the ability to bind antigen. The antibody can be polyclonal, monoclonal, recombinant, chimeric, de-immunized or humanized, fully human, non-human, (e.g., murine), or single chain antibody. In some embodiments, the antibody has effector function and can fix complement. In some embodiments, the antibody has reduced or no ability to bind an Fc receptor. For example, the antibody can be an isotype or subtype, an antibody fragment or mutant, which does not support binding to an Fc receptor, e.g., it has a mutagenized or deleted Fc receptor binding region. The term antibody also includes an antigen-binding molecule based on tetravalent bispecific tandem immunoglobulins (TBTI) and/or a dual variable region antibody-like binding protein having cross-over binding region orientation (CODV).

The terms "fragment" or "antibody fragment" refer to a polypeptide derived from an antibody polypeptide molecule (e.g., an antibody heavy and/or light chain polypeptide) that does not comprise a full-length antibody polypeptide, but that still comprises at least a portion of a full-length antibody polypeptide that is capable of binding to an antigen. Antibody fragments can comprise a cleaved portion of a full length antibody polypeptide, although the term is not limited to such cleaved fragments. Antibody fragments that are useful in the present invention include, for example, Fab fragments, F(ab')2 fragments, scFv (single-chain Fv) fragments, linear antibodies, monospecific or multispecific antibody fragments such as bispecific, trispecific, tetraspecific and multispecific antibodies (e.g., diabodies, triabodies, tetrabodies), monovalent or multivalent antibody fragments such as bivalent, trivalent, tetravalent and multivalent antibodies, minibodies, chelating recombinant antibodies, tribodies or bibodies, intrabodies, nanobodies, small modular immunopharmaceuticals (SMIP), binding-domain immunoglobulin fusion proteins, camelized antibodies, and VHH containing antibodies. Additional examples of antigen-binding antibody fragments are known in the art.

The terms "Complementarity-determining region" or "CDR" refer to short polypeptide sequences within the variable region of both heavy and light chain polypeptides that are primarily responsible for mediating specific antigen recognition. The term "framework region" refers to amino acid sequences within the variable region of both heavy and light chain polypeptides that are not CDR sequences, and are primarily responsible for maintaining correct positioning of the CDR sequences to permit antigen binding. Although the framework regions themselves typically do not directly participate in antigen binding, as is known in the art, certain residues within the framework regions of certain antibodies can directly participate in antigen binding or can affect the ability of one or more amino acids in CDRs to interact with antigen.

Examples of antibodies are anti PCSK-9 mAb (e.g., Alirocumab), anti IL-6 mAb (e.g., Sarilumab), and anti IL-4 mAb (e.g., Dupilumab).

Pharmaceutically acceptable salts of any API described herein are also contemplated for use in a drug or medicament in a drug delivery device. Pharmaceutically acceptable salts are for example acid addition salts and basic salts.

Those of skill in the art will understand that modifications (additions and/or removals) of various components of the APIs, formulations, apparatuses, methods, systems and embodiments described herein may be made without departing from the full scope and spirit of the present invention, which encompass such modifications and any and all equivalents thereof.

It will be further apparent to those skilled in the art that various modifications and variations can be made to the present disclosure without departing from the scope of the disclosure. Further, it is to be noted, that any reference numerals used in the appended claims are not to be construed as limiting the scope of the disclosure.

### Brief description of the drawings

In the following, nonlimiting examples of a fluid dispensing device and of a container for use inside a dispensing device are illustrated in greater detail by making reference to the drawings, in which:
Fig. 1 shows an example of a fluid dispensing device with a container assembled therein in an initial configuration,
Fig. 2 illustrates the device according to Fig. 1 during or after dispensing of a dose of the liquid absence,
Fig. 3 illustrates an isolated container in the initial configuration,
Fig. 4 shows an enlarged view of a portion of the container of Fig. 3,
Fig. 5 shows a further embodiment of a discharge assembly of a fluid dispensing device and/or of a container along a longitudinal cross-section,
Fig. 6 shows the first member of the discharge assembly surrounded or sheathed by the tubular shaped seal,
Fig. 7 schematically shows a spring biased arrangement of a base to the housing of the fluid dispensing device,
Fig. 8 schematically illustrates the working principle of an inlet valve of the fluid dispensing device in an initial configuration,
Fig. 9 shows the valve according to Fig. 8 during a unidirectional flow of a liquid substance,
Fig. 10 shows another example of an outlet valve in an initial configuration,
Fig. 11 shows the valve according to Fig. 10 during a unidirectional flow of a liquid substance,
Fig. 12 shows another example of an outlet valve for use with the fluid dispensing device in an initial and hence sealing configuration,
Fig. 13 shows the valve of Fig. 12 during a unidirectional flow of the liquid substance, and
Fig. 14 shows another example of an inlet and outlet valve of a fluid dispensing device 10.

### Detailed Description

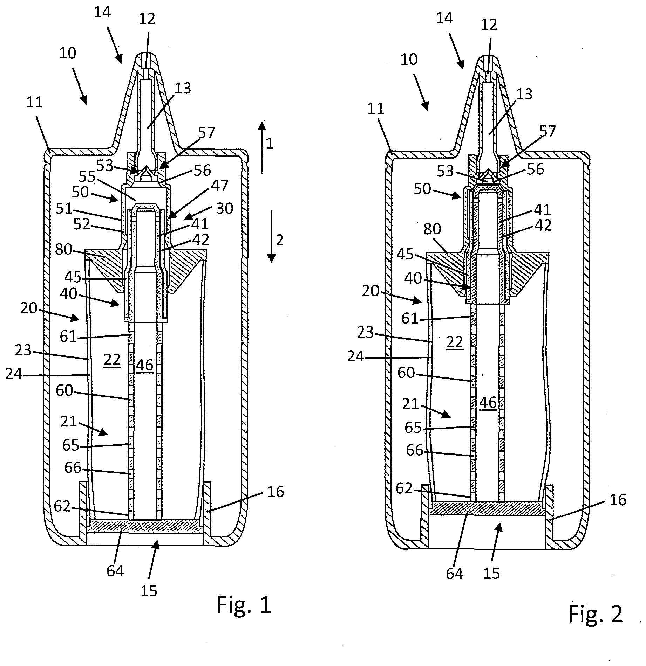

In Figs. 1-4 and example of a fluid dispensing device 10 is illustrated. The fluid dispensing device 10 comprises a housing 11. The housing 11 comprises a tipped applicator section 14 protruding outwardly from the housing 11. As illustrated the housing 11 may comprise a rectangular or cubic shape.

Optionally, the housing 11, in particular the tipped applicator section 14 thereof, may be covered by a detachable protective cap (not illustrated). The housing 11 comprises a rigid structure. The tipped applicator section 14 may be sized and shaped to enter a nostril of a person or patient. At a distal end, hence at an upper end of the tipped applicator section 14 there is provided a spray nozzle 12. The spray nozzle 12 serves to atomize a liquid substance 21 provided through a discharge channel 13 in flow communication with the spray nozzle 12 and located upstream of the spray nozzle 12.

The fluid dispensing device 10 further comprises a container 20. The container 20 comprises a cavity 22 that is confined by a shell 23. The shelf 23 comprises at least a flexible shell portion 24. Typically, the flexible shell portion 24 is elastically and/or plastically deformable. The container 20 may be filled with a liquid substance 21. The liquid substance 21 may contain a pharmaceutically active component. The fluid dispensing device 10 further comprises a discharge assembly 30. The discharge assembly 30 is configured and operable to discharge a well-defined portion of the liquid substance, e.g. a dose of predefined size via the discharge channel 13 towards and through the spray nozzle 12.

The discharge assembly 30 comprises a first member 40 and a second member 50. The first member 40 and the second member 50 are movable relative to each other along a first direction 1 and/or along a second direction 2 in order to discharge a dose of the liquid substance 21 from the cavity 22. The second direction 2 is opposite to the first direction 1. With the present example the first member 40 is displaceable relative to the second member 50 along the first direction 1 for dispensing of a dose of the liquid substance. The first member 40 is displaceable relative to the second member 50 along the second direction 2 for returning into an initial configuration.

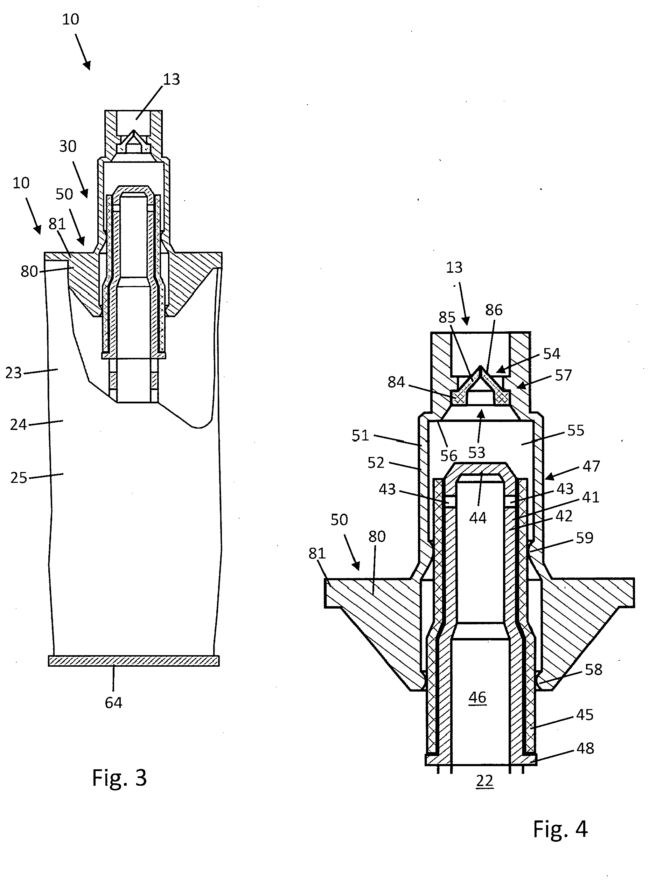

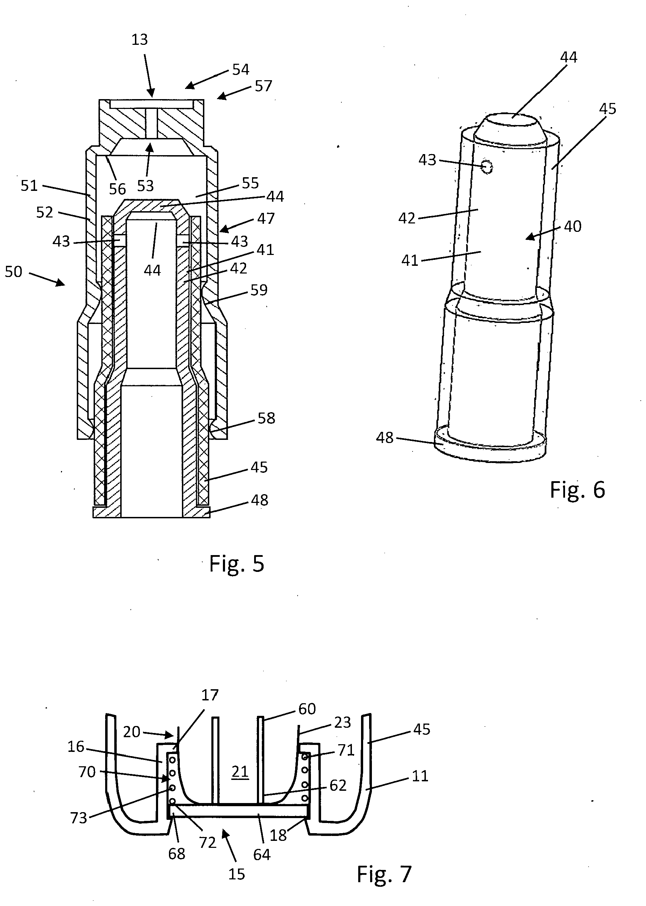

In Fig. 2, the discharge assembly 30 is illustrated during or after dispensing of a dose of the liquid substance 21. Here, compared to the initial configuration as illustrated in Fig. 1 the first member 40 has been moved relative to the second member 50 along the first direction 1, which, in the present example coincides with a longitudinal distal direction. As illustrated in greater detail in Figs. 4 and 5 the first member 40 comprises a hollow tubular section 41 that is in flow communication with the cavity 22. The hollow tubular section 41 of the first member 40 is slidably received inside a hollow tubular section 51 of the second member 50. Accordingly, the hollow tubular section 51 of the second member is open towards the second direction or proximal direction 2 in order to slidably receive a distal end of the hollow tubular section 41 of the first member 40.

As further illustrated in Figs. 4 -6, the tubular section 41 of the first member 40 comprises a tubular-shaped sidewall 42. Inside the sidewall 42 there is provided at least one through opening 43. The through opening 43 provides a flow communication between the interior of the hollow tubular section 41 of the first member 40 and an outside of the first member 40. As shown in Fig. 4, there may be provided numerous through openings 42 extending through the tubular-shaped sidewall 42. Numerous through openings 43 may be distributed around the outer circumference of the tubular section 41 or of the tubular-shaped sidewall 42.

An axial end, typically a distal end of the hollow tubular section 41 of the first member 40 is axially confined by a closed wall 44. The closed wall 44 forms a distal end of the tubular section 41 of the first member 40. In this way, the liquid substance 21 located inside the cavity 22 and being in flow communication with the interior 46 of the tubular section 41 of the first member 40 is only allowed to escape from the interior 46 of the hollow tubular section 41 via the at least one through opening 43 provided in the tubular-shaped sidewall 42. As becomes particularly apparent from Figs. 4 - 6, the tubular-shaped sidewall 42 is sheathed by a flexible tubular shaped seal 45. The seal 45 is made of an elastic material, such as natural or synthetic rubber. It may comprise a polymeric material. The flexible tubular shaped seal 45 may comprise a rather closed tubular-shaped structure. However, it is open towards the distal end. The tubular shaped seal 45 may be tightly fitted around the outer circumference of the tubular-shaped sidewall 42 of the tubular section 41 of the first member 40. The tubular shaped seal 45 covers and closes the at least one through opening 43 of the tubular-shaped sidewall 42. In this way, a kind of a passive self-closing inlet valve 47 for the discharge assembly 30 can be provided. The flexible tubular shaped seal may be at least slightly stretched in radial direction so as to fit over the outside circumference of the tubular section 41 of the first member 44.

As becomes immediately apparent from a comparison of Figs. 1 and 2 the tubular section 51 of the second member 50 is axially confined in distal direction by an end wall 56. Through the end wall 56 there extends an outlet 53 that is in flow communication with the discharge channel 13. In or across the outlet 53 there is provided an outlet valve 57. The outlet valve 57 is configured or implemented as a unidirectional check valve 54. Hence, the outlet valve 57 only allows and supports dispensing of the liquid substance 21 from the interior of the tubular section 51 of the second member 50 into and towards the discharge channel 13.

In the initial configuration as illustrated in Figs. 1 and 4 there is formed or provided a dispensing chamber 55 between the end wall 56 of the second member 50 and the closed wall 44 of the first member 40. The dispensing chamber 55 is confined in radial direction by the sidewall 52 of the tubular section 51 of the second member 50. As the tubular section 41 of the first member 40 is moved in longitudinal direction relative to and inside the hollow tubular section 51 of the second member 50 the size of the dispensing chamber 50 can be varied accordingly. For dispensing of a liquid substance 21 located inside the dispensing chamber 55 through the outlet 53 the tubular section 41 of the first member 40 is moved in longitudinal direction, e.g. along the first direction 1 towards the distal end of the housing 11.

Since the outside of the tubular section 41 of the first member 40 is in sealed engagement with an inside of the tubular section 51 of the second member 50 the distally directed sliding movement of the first member 40 relative to the second member 50 leads to a pressure build-up inside the dispensing chamber 50. A liquid substance 21 located inside the dispensing chamber 55 will then be urged through the outlet 53 and through the outlet valve 57 into and through the discharge channel 13 so as to become atomized when further urged or expelled through the spray nozzle 12.

As illustrated in Fig. 4, the outlet valve 57 comprises a base portion 84 assembled and fixed inside or to the second member 50. The outlet valve 57 further comprises at least a first and a second flap section 85, 86 that may be integrally formed with the base portion 84. As illustrated in Fig. 4, the flap sections 85, 86 extend at a predefined angle in a downstream direction, hence towards the discharge channel 13. The downstream end of the downstream end of the flap sections 85, 87 are located radially inwardly compared to the base portion 84. In this way and as a pressure inside the upstream dispensing chamber 55 exceeds a predefined threshold the skewed or tilted orientation of the flap sections 85, 86 leads to a radially outwardly directed movement of the radially inwardly located ends of the flap sections 85, 86, thus giving way for the liquid substance 21 to pass through the outlet valve 57. In the opposite configuration and when a pressure inside the upstream dispensing chamber 55 should be lower than a pressure downstream of the outlet valve 57, such a pressure will tend to bring the inner ends of the flap sections 85, 86 closer and/or tighter together, thus increasing the sealing capability and sealing effect of the outlet valve.

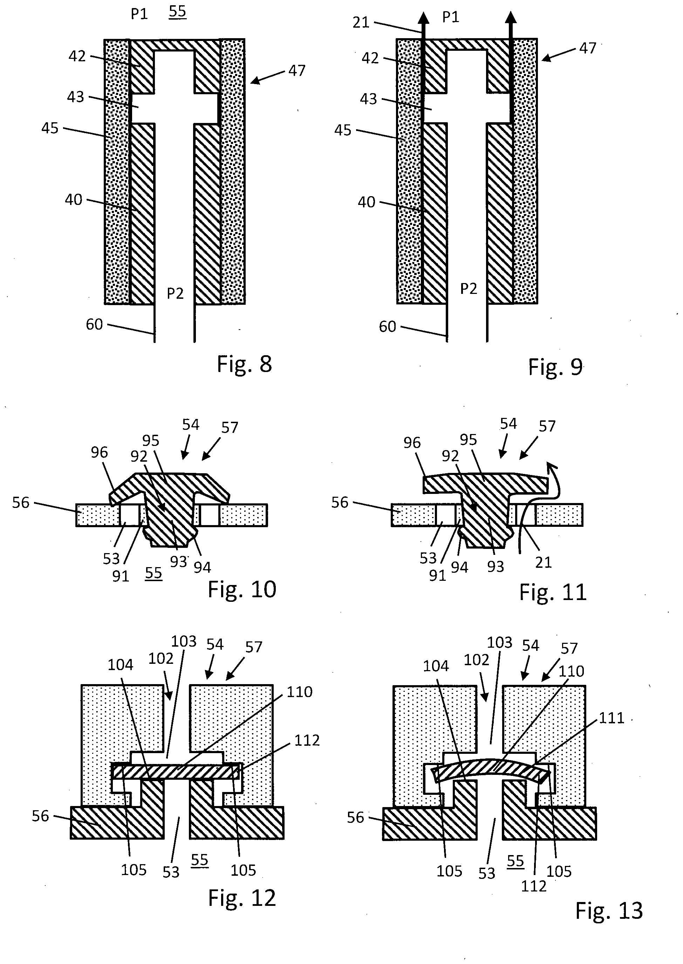

Generally, there may be provided a large variety of outlet valves 57, such as illustrated in Figs. 10-13. for such modified outlet valves 57 the second member 50 may be geometrically adapted, such as indicated in Fig. 5.

As illustrated in greater detail in Fig. 4, on the inside of the sidewall 52 of the tubular section 51 of the second member 50 there are provided annular protrusions 58, 59. The annular protrusion 59 is located at a predefined axial or longitudinal distance from the annular protrusion 58. Both annular protrusions 58, 59 are in direct sealing engagement with the tubular shaped seal 45 surrounding the outer circumference of the tubular section 41 and hence of the tubular sidewall 42 of the first member 40. In this way, the assembly of the first tubular section 41 and the tubular shaped seal 45 is and remains in e.g. permanent sealing engagement with the interior of the tubular section 51 of the second member 50. Typically, the annular protrusions extend uninterrupted all over the inner circumference of the tubular section 51 of the second member 50.

Accordingly and when the first member 40 is subject to a movement along the second direction 2, hence from the dispensing configuration as illustrated in Fig. 2 towards the initial configuration as illustrated in Fig. 1 there may arise a negative pressure inside the increasing dispensing chamber 55. Since the outlet 53 is effectively sealed by the valve 57 and since the sliding engagement between the second member 50 and the first member 40 is leakage-proof there will evolve a negative pressure inside the dispensing chamber 55. The negative pressure inside the dispensing chamber 55 is at a pressure level that is lower than a pressure level inside the cavity 22, which is in flow communication with the interior of the tubular section 41 of the first member 40.

Due to this pressure gradient the liquid substance 21 located inside the hollow tubular section 41 of the first member 40 is withdrawn through the through opening 43 of the sidewall 42 and flows between the outside surface of the sidewall 42 and an inside surface of the flexible tubular shaped seal 45 until it reaches and seals the dispensing chamber 55. When a pressure equilibrium has been reached between the dispensing chamber 55 and the interior of the tubular section 41, a respective flow of the liquid substance 21 from the interior of the tubular section 41 towards the dispensing chamber 55 will stop.

Since the flexible tubular shaped seal 45 covering and effectively closing the through opening 43 in the sidewall 42 the tubular shaped seal 45 forms a kind of a passive self-closing inlet valve 47 providing a unidirectional flow of the liquid substance 21 from the cavity 22 towards and into the dispensing chamber 55. An increase of the liquid pressure inside the dispensing chamber 55 up to a level above the pressure level inside the cavity does not lead to a substantive leakage or backflow of the liquid substance from the dispensing chamber 55 towards and into the hollow tubular section 41.

It should be further noted, that the radially inwardly extending protrusions 58, 59 each may serve as or may constitute a sealing lip providing a smooth sliding displacement of the tubular section 41 inside the tubular section 51. The two protrusions 58, 59 provide the benefit that the assembly of the tubular section 41 and the surrounding seal 45 is mechanically supported at least twice, thus providing a tilt free sliding displacement of the tubular section 41 inside the tubular section 51. Moreover, with the protrusions 58, 59, e.g. implemented as an annular protruding portion of limited size in longitudinal direction a smooth sliding displacement between the tubular section 41 and the tubular section 51 can be obtained.

As further shown in Fig. 4, the proximal end of the flexible tubular shaped seal 45 is in abutment with a radially outwardly extending flange portion 48 at the proximal end of the first member 40. In this way, at least a unidirectional axial abutment can be provided between the tubular section 41 and the flexible tubular shaped seal 45. A distal end of the seal 45 is located close to the distal end face, hence close to the closed wall 44 of the tubular section 41. As illustrated in Fig. 4, the closed wall 44 and hence the distal end of the tubular section 41 slightly protrudes from the distal end of the flexible tubular shaped seal 45. In this way, an unobstructed flow of the liquid substance 21 through the inlet valve 41 can be provided.

The at least one through opening 43 or several circumferentially distributed through openings 43 extending through the tubular-shaped sidewall 42 of the first member 40 is/are typically located in close vicinity to the distal end of the flexible tubular shaped seal 45. In this way, the inlet valve 47 may provide a respective flow of the liquid substance 21 even at a comparatively low pressure gradient between the dispensing chamber 55 and the interior of the hollow tubular section 41.

As it is further apparent from Figs. 1-4, a proximal end of the first member 40 is located inside the cavity 22 of the container 20. As illustrated in Fig. 1, the cavity 22 is confined by a flexible shell 23. The flexible shell 23 comprises at least one or numerous flexible shell portions 24. For instance, the flexible shell portion 24 may comprise a flexible foil 25. As further indicated in Fig. 1 the second member 50 is connected to the shell 23 or forms part of the shell 23. Here, the second member 50, e.g. made of a rigid material comprises a shoulder portion 80 extending radially outwardly from a proximal end section of the tubular section 51 of the second member 50.

The shoulder portion 80 may comprise a somewhat circular or disc like shape extending radially outwardly from the tubular section 51 of the second member 50. Here, an outer rim or a rim portion 81 of the shoulder portion 80 can be connected to the flexible shell portion 24 of the container 20. The radially outwardly located rim portion 81 may be integrally formed with the flexible shell portion. When the shoulder portion 80 and the flexible shell portion 24 should be made of different materials the flexible shell portion 24 may be connected or fixed to the rim portion in a sealed manner. For instance, the rim portion 81 and the flexible shell portion 24 may be mutually bonded or welded to provide an air- and/or liquid-proof seal. The shoulder portion 18 may form or constitute a first longitudinal end of the shell 23.

At an oppositely located second longitudinal end of the shell 23 or container 20 there may be provided a base 64. The base 64 may also comprise a rigid material. It may be made of a plastic material, e.g. a thermoplastic injection moldable material. The base 64 may be slidably guided inside a guiding structure 15 of the housing 11 as illustrated in Fig. 7. For this, the guiding structure 15 may comprise a recessed portion in a sidewall of the housing 11. The guiding structure 15 may comprise a sidewall 16 extending from the sidewall of the housing 11 into the interior of the housing 11. An inner end of the sidewall 16 of the guiding structure 15 may be terminated by a further inwardly extending flange 17 that provides an abutment for a mechanical biasing member 70.

The mechanical biasing member 70 comprises a compression spring 73 having a first end 71 in longitudinal or axial abutment with the flange 17. The compression spring 73 further has a second end 72 opposite the first end 71. The second end 72 is in engagement or in longitudinal abutment with the base 64. In this way, the base 64 is movable from an initial configuration as illustrated in Fig. 1 into a depressed or inwardly shifted position or configuration as illustrated in Fig. 2 against the restoring action of the compression spring 73.

The base 64 comprises an outer edge or a rim portion 68, which is in engagement or in abutment with the mechanical biasing member 70. Additionally and as illustrated in Figs. 1 and 2 the outer edge or rim portion 68 is connected to or fixed to the flexible shell portion 24 at the second end of the container 20. The base 64 may be connected to the flexible shell portion 24 in the same manner as the shoulder portion 80. It may be welded or bonded to the flexible shell portion 24. The base 64 integrated into the shell 23 may form or constitute a second longitudinal end of the shell 23 and hence of the container 20.

As further illustrated in Figs. 1 and 2, the base 64 is rigidly connected to a dip tube 60. The dip tube 60 comprises a first longitudinal end 61 connected to and fixed to the first member 40. An interior of the dip tube 60 is in flow communication with an interior 46 of the hollow tubular section 41 of the first member 40. The dip tube 60 further comprises an opposite, hence a second longitudinal end 62 rigidly connected and fixed to the base 64. The dip tube 60 is made of a rigid material and the dip tube is rigid in compression as seen in longitudinal direction, hence as seen along the first or second directions 1, 2, respectively.

The dip tube 60 comprises a sidewall 65, e.g. of tubular shape. The sidewall 65 may comprise a circular or an elliptical diameter or cross-section. The sidewall 65 of the dip tube 60 may comprise numerous through openings 66 distributed all across the sidewall 65 of the dip tube 60.

Typically, the base 64 is accessible from outside the housing 11 of the fluid dispensing device 10. The base 64 is rigidly connected to the first member 40 via the dip tube 60. In this way, a user may induce a distally directed movement of the first member 40 relative to the second member 50 by applying an inwardly directed pressure onto the base 64. The second member 50 is typically fixed inside the housing 11 of the fluid dispensing device 10.

A user aiming to produce a spray discharge of the liquid substance 21 through the discharge channel 13 and hence through the spray nozzle 12 may urge the base 64 into the housing 11, typically along the first direction 1. The movement of the base 64 is transferred to the dip tube 60 and hence to the first member 40, all of which being rigidly connected to each other. Since the second member 50 is rigidly connected to the housing 11 the longitudinal sliding displacement of the base 64 relative to the housing 11 induces a distally directed movement of the first member 40 relative to the second member 50. A liquid substance 21 located inside the dispensing chamber 55 will be urged through the outlet valve 57 into the discharge channel 13 and through the spray nozzle 12. Consequently, a spray or an atomized cloud of an aerosol will be discharged from the spray nozzle 12.

The distally directed displacement of the base 64 relative to the housing 11 comes along with energy accumulation in the mechanical biasing member 70. When a user releases the base 64, the mechanical biasing member 70 releases previously stored mechanical energy and serves to return the base 64 into the initial configuration as shown in Fig. 1. The proximally directed movement of the base 64 is stopped by a stop face 18 of the housing, which stop face at least slightly protrudes inwardly from the sidewall 16 of the guiding structure 15. For the purpose of a smooth assembly of the base 64 into the guiding structure 15 of the housing 11 the radially inwardly facing sidewall of the radially inwardly protruding stop faces 18 comprises a beveled section 19.

The return motion of the base 64 into the initial position or initial configuration as provided by the mechanical biasing member 70 equally transfers to the dip tube 60 and to the tubular section 41 of the first member 40. Accordingly, and due to the proximally directed movement of the first member 40 relative to the second member 50, the size and volume of the dispensing chamber 55 will increase again. Due to the sealing engagement of the first and second members 40, 50 a negative pressure will build up inside the dispensing chamber 55 thus leading to a repeated ingress of the liquid substance 21 into the dispensing chamber 55.

Since the shell 23 of the container 20 is at least in portions flexible, ingress of the liquid substance 21 into the dispensing chamber 55 is accompanied by at least a slight inwardly directed deformation of the flexible shell portion 24. Typically, the flexible shell portion is plastically deformable rather than elastically deformable. The flexible shell portion 24 does not provide or generate substantial restoring forces. Moreover, the cavity 22 may be entirely filled with the liquid substance, e.g. with a liquid medicament effectively free of any gaseous components. In this way a highly effective withdrawal of the liquid substance 21 from the cavity 22 into the dispensing chamber 55 and further into the discharge channel 13 can be provided irrespective of an orientation of the fluid dispensing device.

Moreover and since the dip tube 60 is perforated all along its longitudinal direction, a rather homogeneous withdrawal of the liquid substance from almost any volumetric portion of the cavity 22 can be provided.

Since the shell 23 is at least in sections flexible it will be subject to a stepwise deformation each time a dose of the liquid substance 21 has been withdrawn from the cavity 22. Due to the plastic deformation capability of the flexible shell portion(s) 24 the cavity 22 can remain gas-less during and/or after repeated dispensing operations thus allowing for an orientation invariant withdrawal of the liquid substance 21 from the cavity 22.

In Figs. 8 and 9 the working principle of the inlet valve 47 is schematically illustrated. In the configuration of Fig. 8, a pressure P2 inside the tubular section 41 of the first member 40 is equal to or smaller than a pressure P1 inside the dispensing chamber 55. As a consequence, and even if the first member 40 should be subject to an axial or longitudinal displacement, thereby reducing the volume of the dispensing chamber 55 the inlet valve 47 will remain closed. A backflow of the liquid substance 21 from the dispensing chamber 55 through a non-existing gap between the outside surface of the tubular-shaped sidewall 42 and an inside surface of the flexible tubular shaped seal 45 can be effectively prevented.

In Fig. 9 another configuration is illustrated, wherein a pressure P2 inside the hollow tubular section 41 and hence inside the sidewall 42 is larger than the pressure inside the dispensing chamber 55. Then and due to the elastic behavior of the flexible tubular shaped seal 45 a streamlet of the liquid substance 21 is allowed to flow through the at least one through opening 43 of the tubular-shaped sidewall 42 and hence through a gap formed between an inside surface of the slightly deformed tubular shaped seal 45 and an outside surface of the tubular-shaped sidewall 42. The flow of the liquid substance 21 automatically stops, when an equilibrium between the pressure P1 and P2 is obtained. This will be typically the case, when the dispensing chamber 55 is effectively or entirely filled with the liquid substance 21. In Fig. 10 and 11 another example of an outlet valve 57 is illustrated. This outlet valve 57 is also implemented as a check valve It may be used instead of the outlet valve 57 as illustrated in Fig. 4.

With the example of Fig. 10, a kind of an umbrella outlet valve 57 is implemented. The outlet valve 57 also comprises and forms a check valve 54. Here, the outlet 53 comprises an annular shaped discharge outlet extending through the end wall 56 of the second member 50. Alternatively, there are provided numerous discharge outlets 53 located radially outwardly with regards to a mount 91 extending through the end wall 56. The end wall 56 comprises a central mount 91 comprising a through opening 92 in which a base portion 93 of the outlet valve 57 is fixed. The base portion 93 comprises a radially outwardly extending bulged portion or a radially outwardly extending protrusion 94 gripping under the proximal side of the end wall 56.

The base portion 93 of the sealing body 95 extends through the through opening of the central mount 92. On a distal side of the end wall 56, the valve body 95 comprises a radially outwardly extending and proximally directed flange section 96. The flange section 96 is oriented radially outwardly and proximally. An outer end of the flange section 96 is in a sealing engagement with a distal side of the end wall 56. The size and the radial extent of the flange section 96 is larger than the diameter or cross-section of the outlet 53 extending through the end wall 56.

In an initial configuration as illustrated in Fig. 10 the flange section 96 of the sealing body 95 effectively seals the outlet 53. If a pressure on the proximal, hence on the upstream side of the outlet valve 57 should be larger than a pressure at a distal and hence at a the downstream side of the outlet valve 57 the increased pressure will lead to a reversible deformation of the flange section 96 as illustrated in Fig. 11. Accordingly, the flange section will become subject to an elastic deformation thereby disengaging from the outlet 53 and giving way for the liquid substance 21 discharging into the discharge channel 13. In the opposite scenario and when the pressure on a downstream side of the outlet valve 57 should be larger than on an upstream side, such an increased pressure will only tend to bring the flange section 96 in a tighter sealing engagement with the distal side of the end wall 56, thereby increasing the sealing capability and sealing functionality of the outlet valve 57. The outlet valve 57 as illustrated in Figs. 10 and 11 may be used instead of the outlet valve 57 as illustrated in Figs. 1-4.

The outlet valve 57 as illustrated in Figs. 10 and 11 and hence the sealing body 95 may be made or provided as a single unitary piece of an elastomeric material, such as natural or synthetic rubber. It may provide excellent sealing capability with a neglectable degree of backflow. Moreover, such a sealing body 95 exhibits a desired long term stability and is available at moderate or low costs.

In Figs. 12 and 13 another example of an outlet valve 57 is schematically illustrated. Here, the outlet valve 57 comprises an elastic disc 110. The elastic or flexible disc 110 is located and supported by a support 104 axially or longitudinally protruding from a distal side of the end wall 56. The support 104 is typically in longitudinal alignment with the outlet 53 extending through the end wall 56. The support 104 may be provided by a cylindrical protrusion extending distally from the distal side of the end wall 56. The support 104 provides a longitudinal abutment for a radial central portion of the flexible disc 110. The flexible sealing disc 110 comprises an outer radial edge 112. The outer edge 112 protrudes radially outwardly from the support 104 of the end wall 56.

The outer edge 112 is in abutment with a further support 105 of a mount 101 connected and fixed to the distal side of the end wall 56. The support 105 provides a distal support for the outer edge 112 of the flexible disc 110. The mount 110 comprises an outlet 102 extending in longitudinal direction through the mount 101. A proximal end of the outlet 102 terminates or merges into a receiving space 103, in which the flexible disc 110 is arranged and mounted.

As illustrated in Fig. 12, the support 105 of the mount 101 is in sealing engagement or is at least in abutment with the distal side of the outer edge 112 of the flexible sealing disc 110. The support 104 of the end wall 56 protruding longitudinally in distal direction from the end wall 56 is in abutment with a proximal side of the flexible disc 110 at a radial distance from the outer edge 112.

In situations, in which a pressure downstream of the flexible sealing disc 110 is larger than upstream of the flexible sealing disc 100, hence in situations where a pressure in the region of the outlet 102 is larger than in the region of the outlet 53, the pressure gradient serves to press the flexible disc 110 in tight or even tighter mechanical engagement with the radial innermost support 104. Ingress of gaseous substances or of impurities from the outlet 102 into the outlet 53 is therefore effectively blocked and prevented.

In the other situation as illustrated in Fig. 13 and when a pressure on the upstream side of the outlet valve 57 is larger than on the downstream side, hence when the pressure in the region of the outlet 53 is larger than in the region of the outlet 102, the flexible disc 110, in particular a central portion thereof, will be subject to a distally directed deformation. Hence, as illustrated in Fig. 13, a radial inner portion of the flexible sealing disc 110 starts to move and to deform into the receiving space 103 of the mount 101.

This pressure-induced local deformation of the flexible sealing disc 110 leads to a counter movement of the outer edge 112 towards the proximal direction because a radial middle portion 111 of the flexible sealing disc 110 is in longitudinal or axial abutment with the support 104. The flexible deformation of the flexible sealing disc 110 then leads to a disengagement of the outer edge 112 of the flexible sealing disc 110 from both, the distally facing support 104 previously in engagement with a proximal side of the sealing disc and from the proximally facing support 105 previously in sealing engagement with a distal side of the outer edge 112 of the flexible sealing disc 110.

Consequently, a flow of the liquid substance 21 from the interior of the dispensing chamber 55 towards and into the outlet 102 and hence into the discharge channel 13 will be provided and supported.

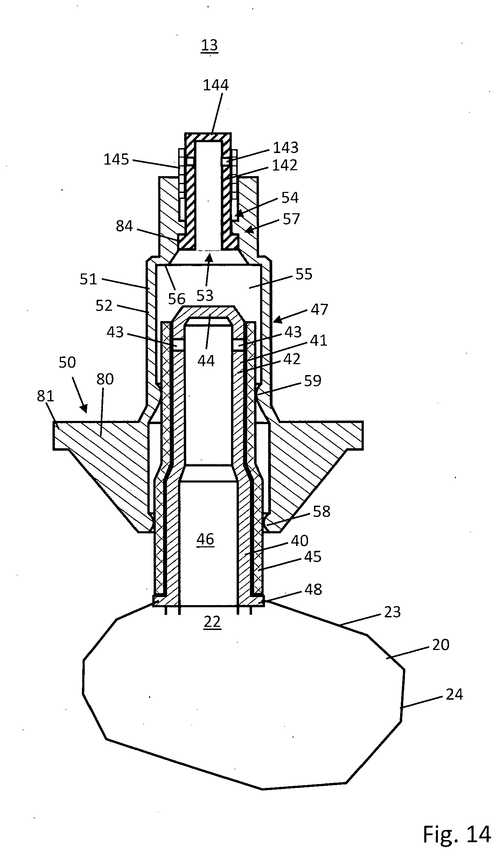

In Fig. 14, another example of the fluid dispensing device 10 is illustrated. Here, and contrary to the example of Figs. 1-4 the container 20 is connected and fixed to the first member 40. The tubular section 41 and hence the sidewall 42 of the first member 40 may reach through or may penetrate the shell 23 of the container 20. The first member 40 may be in sealing engagement with the shell 23. In this way, the interior 46 of the first member 40 is in direct and permanent fluid communication with the cavity 22 of the container 20.

With the example of Fig. 14, the outlet 53 of the fluid dispensing device 10 is sealed by an outlet valve 157 implemented as a one-way valve 154. Basically, the outlet valve 157 comprises the same construction and follows the same working principle as the inlet valve 47. Regarding the shape, construction and working principle of the outlet valve 157, reference can be made to the inlet valve 47, accordingly. Hence, all features, benefits and effects as described in connection with the inlet valve 47 equally apply to the outlet valve 157.

The outlet valve 157 comprises a tubular part or section 141, which is axially confined by a closed wall 144 towards the discharge channel 13. The tubular section 41 and hence the tubular-shaped sidewall 142 is sheathed by a flexible tubular shaped seal 145. There are further provided at least one or numerous through openings 143 extending radially through the sidewall 142. The seal 145 effectively seals the through openings 143.

In the event that a pressure inside the interior 146 of the tubular section 141 is larger than in the adjacent discharge channel 13 the liquid substance contained in the dispensing chamber 55, which is in permanent fluid communication with the interior 146, is expelled through the through openings 143, thereby at least temporally and radially outwardly deforming the tubular seal 143.

### List of reference numbers

- 1: distal direction
- 2: proximal direction
- 10: fluid dispensing device
- 11: housing
- 12: spray nozzle
- 13: discharge channel
- 14: applicator section
- 15: guiding structure
- 16: sidewall
- 17: flange
- 18: stop face
- 19: beveled section
- 20: container
- 21: liquid substance
- 22: cavity
- 23: shell
- 24: flexible shell portion
- 25: flexible foil
- 30: discharge assembly
- 40: first member
- 41: tubular section
- 42: sidewall
- 43: through opening
- 44: closed wall
- 45: seal
- 46: interior
- 47: inlet valve
- 48: flange portion
- 50: second member
- 51: tubular section
- 52: sidewall
- 53: outlet
- 54: check valve
- 55: dispensing chamber
- 56: end wall
- 57: outlet valve
- 58: protrusion
- 59: protrusion
- 60: dip tube
- 61: longitudinal end
- 62: longitudinal end
- 64: base
- 65: sidewall
- 66: through opening
- 68: rim portion
- 70: mechanical biasing member
- 71: first end
- 72: second end
- 73: spring
- 80: shoulder portion
- 81: rim portion
- 84: base portion
- 85: flap section
- 86: flap section
- 91: mount
- 92: through opening
- 93: base portion
- 94: protrusion
- 95: sealing body
- 96: flange section
- 101: mount
- 102: outlet
- 103: receiving space
- 104: support
- 105: support
- 110: flexible disc
- 111: middle portion
- 112: outer edge
- 141: tubular section
- 142: sidewall
- 143: through opening
- 144: closed wall
- 145: seal
- 146: interior
- 154: one-way valve
- 157: outlet valve

## Claims

1. A fluid dispensing device (10) comprising:
- a housing (11) sized to accommodate a container (20) filled with a liquid substance (21),
- the container (20) comprising a cavity (22) confined by a shell (23), the shell (23) comprising at least a flexible shell portion (24),
- a discharge assembly (30) comprising a first member (40) and a second member (50),
- the first member (40) comprising a hollow tubular section (41) in flow communication with the cavity (22), and
- the first member (40) and the second member (50) being movable relative to each other along a first direction to discharge a dose of the liquid substance (21) from the cavity (22).

2. The fluid dispensing device (10) according to claim 1, wherein the second member (50) comprises a hollow tubular section (51) sized to slidably receive the hollow tubular section (41) of the first member (40) therein.

3. The fluid dispensing device (10) according to any one of the preceding claims, wherein the tubular section (41) of the first member (40) comprises a tubular-shaped sidewall (42) with at least one through opening (43).

4. The fluid dispensing device (10) according to any one of the preceding claims, wherein the tubular section (41) of the first member (40) is axially confined by a closed wall (44)

5. The fluid dispensing device (10) according to any one of the preceding claims, wherein the tubular section (41) of the first member (40) is sheathed by a flexible tubular shaped seal (45).

6. The fluid dispensing device (10) according to any one of the preceding claims, wherein the tubular section (41) of the first member (40) is in sealing engagement with the tubular section (51) of the second member (50).

7. The fluid dispensing device (10) according to any one of the preceding claims, wherein the hollow tubular section (51) of the second member (50) comprises a dispensing chamber (55) axially confined by the tubular section (41) of the first member (40), wherein the size of the dispensing chamber (55) is variable by moving the first member (40) relative to the second member (50).

8. The fluid dispensing device (10) according to any one of the preceding claims, wherein the second member (50) comprises an outlet (53) at a longitudinal end of the hollow tubular section (51) of the second member (50) that faces away from the first member (40), wherein the outlet (53) is sealed by a check valve (54) or one-way valve (154).

9. The fluid dispensing device (10) according to any one of the preceding claims, wherein the shell (23) comprises a flexible foil (25) or is made of a flexible foil (25).

10. The fluid dispensing device (10) according to any one of the preceding claims, wherein an interior (46) of the hollow tubular section (41) of the first member (40) is in flow communication with a dip tube (60) extending into the cavity (22) of the container (20).

11. The fluid dispensing device (10) according to claim 10, wherein the dip tube (60) comprises a first longitudinal end (61) and a second longitudinal end (62) opposite the first longitudinal end (61), wherein the first longitudinal end (61) is connected to the first member (40).

12. The fluid dispensing device (10) according to claim 11, wherein the dip tube (60) is pressure resistant at least with regard to the first direction and wherein the second longitudinal end (62) of the dip tube (60) is connected to a base (64) or is operable engaged, wherein the base (64) is movable relative to at least one of the housing (11) and the second member (50) along the first direction.

13. The fluid dispensing device (10) according to claim 11 or 12, wherein the dip tube (60) comprises a sidewall (65) perforated by numerous through openings (66).

14. The fluid dispensing device (10) according to any one of the preceding claims, further comprising a mechanical biasing member (70) operably engaged with two of the first member (40), the second member (50) and the housing (11), wherein the first member (40) being movable relative to the second member (50) along the first direction against a restoring force provided by the mechanical biasing member (70).

15. The fluid dispensing device (10), wherein the mechanical biasing member (70) comprises a compression spring (73) having a first end (71) and a second end (72) opposite the first end (71), wherein the first end (71) is in abutment with the housing (11) and wherein the second end (72) is mechanically engaged with or is in mechanical abutment with the first member (40) or the second member (50).
